# EUROPÄISCHE PATENTSCHRIFT

(11) **EP 1 363 690 B1**
(45) Veröffentlichungstag und Bekanntmachung des Hinweises auf die Patenterteilung: **28.06.2006**
(21) Anmeldenummer: 02719930.6
(22) Anmeldetag: 26.02.2002
(51) Int. Cl.: A61M 5/32

(54) **SCHUTZVORRICHTUNG FÜR EINE INJEKTIONSNADEL**
PROTECTIVE DEVICE FOR A HYPODERMIC NEEDLE
DISPOSITIF DE PROTECTION D'UNE AIGUILLE DE SERINGUE

(30) Priorität: 26.02.2001 DE 20103363 U
(43) Veröffentlichungstag der Anmeldung: 26.11.2003
(73) Patentinhaber: B. BRAUN MELSUNGEN AG, 34212 Melsungen (DE)
(72) Erfinder: WOEHR, Kevin, 34587 Felsberg (DE); FUCHS, Jürgen, 34308 Bad Emstal (DE)
(74) Vertreter: Klingseisen, Franz
(86) Internationale Anmeldenummer: PCT/EP2002/002042
(87) Internationale Veröffentlichungsnummer: WO 2002/068022

(56) Entgegenhaltungen:
- WO-A-00/69501
- WO-A-99/08742
- US-A- 5 344 408

## Beschreibung

Die Erfindung betrifft eine Schutzvorrichtung für eine Injektions- bzw. Infusionsnadel nach dem Oberbegriff des Anspruchs 1.

Eine Vorrichtung dieser Art ist aus WO-A-99/08742 bekannt, wobei das Griffteil durch einen hohlen Katheteransatz gebildet ist, dessen distales Ende mit einem Katheter verbunden ist. Die gegenüberliegenden Federarme des Schutzelementes greifen in Nuten am Katheteransatz ein und werden im Katheteransatz gehalten, bis die Nadel soweit zurückgezogen ist, dass die Nadelspitze hinter die abgewinkelten Enden der Federarme zu liegen kommt, worauf die Federarme sich vom Katheteransatz lösen und die Nadelspitze abdecken. Die Nadel mit Schutzelement wird danach vollständig vom Katheteransatz gelöst, wobei das Schutzelement an der Nadelspitze freiliegt.

US-A-5,344,408 beschreibt eine Schutzvorrichtung für eine Injektionsnadel mit einer inneren, die Nadel umgebenden Hülse als Schutzelement und einer äußeren Hülse als Griffteil, das lösbar mit dem Schutzelement verbunden ist, so dass die äußere Hülse in distaler Richtung sich vom Schutzelement lösen kann.

Eine weitere Schutzvorrichtung ist aus US 4 929 241 bekannt, wobei ein relativ kleines Schutzelement auf der Nadel angeordnet ist, das mittels einer Feder aus der zurückgezogenen Stellung in die Schutzstellung an der Nadelspitze verschoben werden kann, wobei elastische Arme des Schutzelementes die Nadelspitze übergreifen, während eine Eingriffseinrichtung am Schutzelement dieses auf dem Nadelschaft hält. Aufgrund der geringen Größe des Schutzelementes ist es schwierig, dieses von Hand auf der Nadel zu verschieben. Zudem kann die Befestigungsfeder erst dann ausgelöst werden, wenn die Nadelspitze frei liegt, so dass eine Verletzungsgefahr nicht ausgeschlossen werden kann.

Der Erfindung liegt die Aufgabe zugrunde, eine Schutzvorrichtung der eingangs angegebenen Art so auszubilden, dass eine Betätigung von Hand erleichtert wird und eine Verletzungsgefahr ausgeschlossen werden kann.

Diese Aufgabe wird erfindungsgemäß durch die Merkrnale des Anspruchs 1 gelöst. Dadurch, dass ein Griffteil zum Verschieben des Schutzelementes vorgesehen ist, kann beim Zurückziehen der Nadel mittels des Nadelhalters nach einer Injektion mit der anderen Hand das Griffteil bequem gehalten werden, so dass durch die Relativbewegung zwischen Nadel und Griffteil das Schutzelement in die Schutzposition an der Nadelspitze verschoben wird, ohne dass das kleine Schutzelement mit den Fingern berührt werden muss. Dadurch, dass das Schutzelement während des Zurückziehens der Nadel in die Schutzposition gebracht wird, kann auch eine Verletzungsgefahr ausgeschlossen werden.

Beispielsweise Ausführungsformen der Erfindung werden nachfolgend anhand der Zeichnungen näher erläutert. Es zeigen
- Fig. 1: in einem Längsschnitt eine Schutzvorrichtung,
- Fig. 2: eine Seitenansicht der Ausführungsform nach Fig. 1
- Fig. 3: eine Ansicht der Vorrichtung nach den Fig. 1 und 2 mit in die Schutzstellung verschobenem Schutzelement,
- Fig. 4: eine abgewandelte Ausführungsform der Vorrichtung, und
- Fig. 5: eine Schnittansicht durch eine andere Ausführungsform mit gekrümmter Nadel.

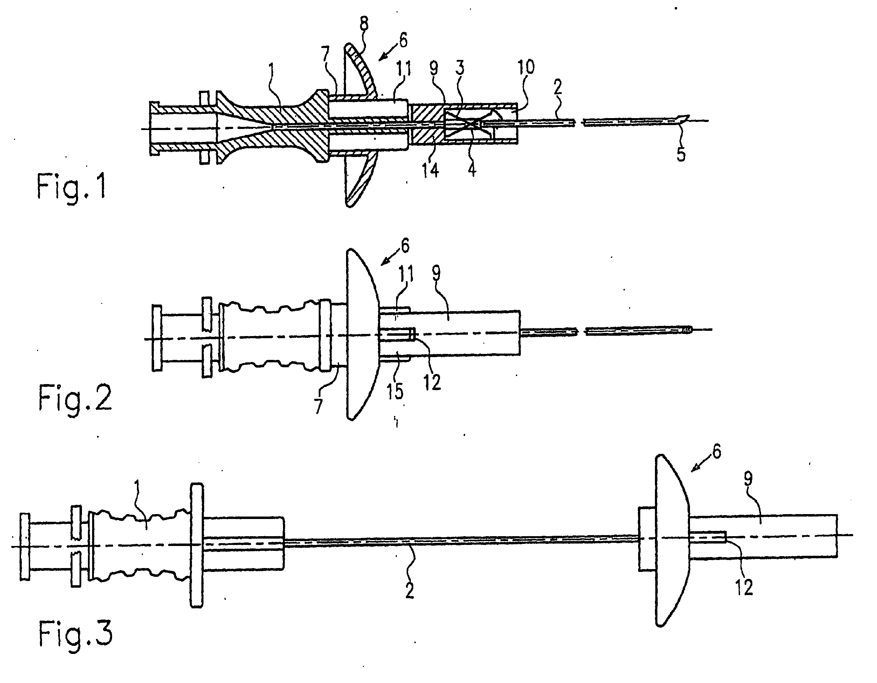

Fig. 1 zeigt einen Nadelhalter 1, in dem eine Nadel 2 befestigt ist. Auf dem Schaft der Nadel 2 ist ein Schutzelement 3 in Form eines Federclips mit sich kreuzenden Armen angeordnet. Mit 4 ist eine Hülse bezeichnet, die mit dem Schutzelement 3 längs des Nadelschaftes verschiebbar ist. Bei dem dargestellten Ausführungsbeispiel ist die Spitze 5 der Nadel entsprechend einer Epidural-Nadel oder einer Huber-Nadel gekrümmt ausgebildet, so dass die Hülse 4, die einen kleineren Durchmesser als die Krümmung an der Nadelspitze aufweist, und mit ihr das Schutzelement 3 nicht über die Nadelspitze hinaus verschoben werden kann.

Zwischen Nadelhalter 1 und Schutzelement 3 ist ein Griffteil 6 angeordnet, das am proximalen Ende einen hohlzylindrischen Abschnitt 7 aufweist, an dem ein radial abstehender Schild 8 ausgebildet ist. Auf der Vorderseite des Schildes 8 ist ein zylindrischer Abschnitt 9 ausgebildet, dessen distales Ende hohl ausgebildet ist. In dem Hohlraum 10 ist in der Bereitstellung nach Fig. 1 das Schutzelement 3 angeordnet, das durch Verschieben des Griffteiles 6 nach vorne zur Nadelspitze 5 verschoben werden kann, während mit der anderen Hand der Nadelhalter 1 gehalten wird. Hierbei übergreifen die abgewinkelten Enden der sich kreuzenden Arme des Schutzelementes 3 die Nadelspitze 5, so dass eine Verletzung der Bedienungsperson durch die Nadelspitze verhindert wird.

Der Nadelhalter 1 weist am distalen Ende radial abstehende Rippen 11 auf, auf denen der hohlzylindrische Abschnitt 7 des Griffteiles 6 geführt ist. Zwischen dem zylindrischen Abschnitt 9 mit kleinerem Außendurchmesser und dem hohlzylindrischen Abschnitt 7 mit größerem Außendurchmessers sind in dem Griffteil 6 Schlitze 12 ausgebildet, durch welche die vorderen Enden der Rippen 11 des Nadelhalters 1 radial vorstehen, wie Fig. 2 zeigt.

Der mit dem Hohlraum 10 versehene zylindrische Abschnitt 9 des Griffteils 6 weist einen vollzylindrischen Abschnitt 14 zwischen den Schlitzen 12 und dem Hohlraum 10 auf, in dessen zentrischer Bohrung die Nadel 2 geführt ist. Zwischen den Schlitzen 12 des Griffteils 6 ist der zylindrische Abschnitt 9 über Stege 15 mit dem Schild 8 bzw. dem hohlrylindrischen Abschnitt 7 einstückig verbunden.

Diese über den Außenumfang des zylindrischen Abschnittes 9 des Griffteiles 6 vorstehenden Rippen 11 dienen zum Aufstecken einer Nadelkappe. Diese Nadelkappe wird für die Lagerung und die Handhabung der Vorrichtung verwendet. Sie kann unmittelbar vor Gebrauch der Injektionsnadel vom Nadelhalter 1 abgezogen werden, um die Nadel freizulegen, ohne dass dadurch das Griffteil 6 und das Schutzelement 3 bewegt wird, weil die Nadelkappe durch die Rippen 11 in einem radialen Abstand von dem Abschnitt 9 des Griffteils 6 gehalten wird.

Aufgrund des kleineren Durchmessers am Abschnitt 14 gegenüber dem größeren Durchmesser an den Rippen 11 kann die Nadelkappe, die aus einem Schlauchstück mit durchgehend gleichem Durchmesser besteht, nicht auf dem Abschnitt 14 fehlpositioniert, sondern nur auf die Rippen 11 aufgesteckt werden. Hierdurch wird verhindert, dass ein Eingriff der Nadelkappe mit einem Abschnitt des Griffteils 6 unbeabsichtigt hergestellt wird. Die Nadelkappe kann durch Extrudieren eines Schlauches kostengünstig gefertigt werden, wobei ein Stück eines solchen Schlauches die Nadelkappe bildet.

Nach Abnehmen der Nadelkappe kann in der Bereitstellung nach den Fig. 1 und 2 eine Injektion ausgeführt werden, worauf beim Zurückziehen der Nadel mit einer Hand am Nadelhalter 1 mit der anderen Hand das Griffteil 6 am Abschnitt 7 gehalten wird, so dass durch die Relativbewegung zwischen Griffteil 6 und Nadel 2 das Schutzelement 3 in die Schutzstellung an der Nadelspitze verschoben wird. Diese ausgefahrene Stellung des Griffteiles 6 ist in Fig. 3 wiedergegeben.

Das Schutzelement 3 ist in dem Hohlraum 10 des Griffteils 6 lose angeordnet, so dass das Griffteil 6 aus der Stellung in Fig. 3 ohne weiteres zurückgezogen werden kann, während das Schutzelement in der Schutzstellung an der Nadelspitze verbleibt. Der Hohlraum 10 im zylindrischen Abschnitt 9 schützt das Schutzelement 3 nach Abziehen der Nadelkappe.

Bei der Ausführungsform nach Fig. 1 und 2 dient der hohlzylindrische Abschnitt 7 am Griffteil 6 dazu, die Finger der das Griffteil haltenden Hand vor einer Berührung mit dem Nadelschaft zu schützen, wenn die Nadel zurückgezogen wird.
Bei einer anderen Ausgestaltung des Nadelhalters 1 kann dieser hohlzylindrische Abschnitt 7 hinter dem Schild 8 vergrößert ausgebildet sein.

Das Griffteil 6 wird - wie auch der Nadelhalter 1- zweckmäßigerweise aus Kunststoff gefertigt.

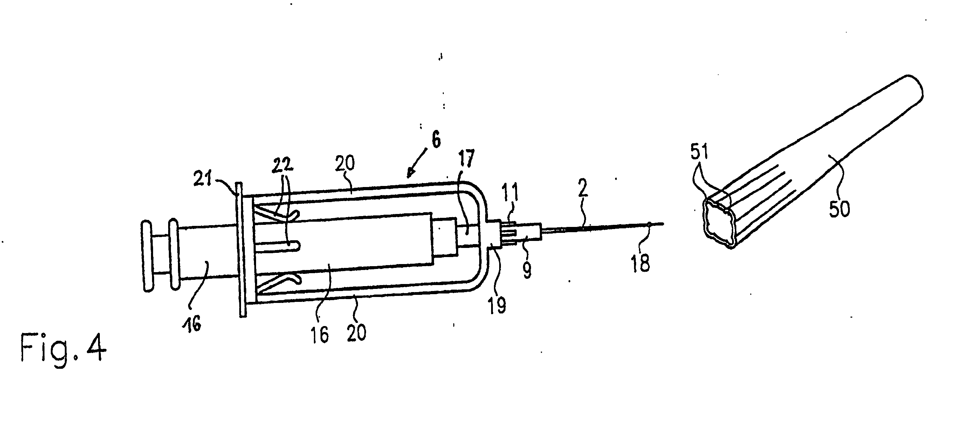

Fig. 4 zeigt eine abgewandelte Ausführungsform eines Griffteils 6 in Verbindung mit einer Spritze 16, an der über einen Nadelhalter 17, der als Kanülenansatz ausgebildet ist, eine Injektionsnadel 2 fest angebracht ist. Bei dieser Ausführungsform ist vor der Nadelspitze ein Wulst 18 auf dem Außenumfang der Nadel ausgebildet, an dem die Rückwand des Schutzelementes 3 in der Schutzstellung zum Anliegen kommt. Anstelle eines Wulstes 18 können durch eine Nadelquetschung auch diametral gegenüberliegende noppenartige Vorsprünge ausgebildet werden.

Das Griffteil 6 weist einen zylindrischen Abschnitt 19 auf, der in der Ausgangsstellung nach Fig. 4 auf dem Nadelhalter 17 geführt ist. Von diesem zylindrischen Abschnitt 19 erstrecken sich bei dem dargestellten Ausfiiluvngsbeispiel an diametral gegenüberliegenden Stellen zwei Bügel 20 in einem Abstand vom Spritzenumfang in proximale Richtung. Die Enden dieser Bügel 20 sind an einem Ringkörper 21 angeformt, von dem aus sich elastische Finger 22 radial nach innen erstrecken. Die freien Enden dieser elastischen Finger 22 liegen auf dem Au-Benumfang der Spritze 16 an.

Aufgrund der elastischen Finger 22 zwischen Griffteil 6 und Außenumfang der Spritze 16 kann das Griffteil 6 für unterschiedlich große Spritzendurchmesser verwendet werden, z. B. können Spritzen mit einem Volumen von 1 ml bis 10 ml in das gleiche Griffteil eingesetzt werden. Hierdurch ist eine große Auswahl von Spritzen möglich, die mit der gleichen Nadel verwendet werden können.

Auch bei der Ausführungsform nach Fig. 4 sind am vorderen Ende des als Kanülenansatz ausgebildeten Nadelhalters radial abstehende Rippen 11 ausgebildet werden, die als Sitz für eine Nadelkappe dienen. Durch den Innenumfang des zylindrischen Abschnitts 19 wird das Schutzelement 3, dessen Rückwand über den Querschnitt der Rippen 11 vorsteht, nach vorne in die Schutzstellung verschoben.

Au dem zylindrischen Abschnitt 19 ist ein weiterer zylindrischer Abschnitt 9 angeformt ist, in dessen Hohlraum 10 das Schutzelement 3 aufgenommen wird. Wie bei der Ausführungsform nach den Fig. 1 und 2 sind zwisehen zylindrischem Abschnitt 19 und Abschnitt 9 in Achsrichtung verlaufende Schlitze ausgebildet, durch die die am Nadelhalter bzw. Kanülenansatz 17 ausgebildeten Rippen 11 zur Aufnahme der Nadelkappe 13 vorstehen.

Fig. 4 zeigt in perspektivischer Ansicht eine durch Spritzgießen ausgebildete Nadelkappe 50, deren distales Ende geschlossen sein kann, während das proximale Ende auf dem Innenumfang entsprechend der Anzahl der Rippen 11 Rillen oder Nuten 51 aufweist, die beim Aufstecken der Nadelkappe auf den Nadelhalter 17 mit den Rippen 11 in Eingriff treten, so dass durch Verdrehen der aufgesteckten Nadelkappe 50 auch der Nadelhalter 17 verdreht werden kann. Zwischen Nadelhalter 17 und Spritze 16 wird üblicherweise ein Gewindeeingriff vorgesehen, so dass durch Verdrehen der Nadelkappe 50 der Nadelhalter 17 auf die Spritze 16 auf geschraubt werden kann.

Es ist üblich, mittels einer Nadel mit relativ großem Durchmesser Flüssigkeit in die Spritze anzusaugen und dann die Nadel gegen eine Nadel mit einem relativ kleinen Durchmesser auszuwechseln um eine Infusion am Patienten auszuführen. Bei der Ausfdbrunisform nach Fig. 4 kann die Nadel ohne weiteres ausgewechselt werden.
Die beschriebene Ausgestaltung ermöglicht eine Betätigung mit einer Hand, wenn der Spritzeninhalt injiziert ist, wobei die Spritze 16 mit zwei Fingern gehalten und die Nadel aus der Haut des Patienten gezogen wird, während gleichzeitig sich ein Finger der Hand an dem am proximalen Ende liegenden Ringkörper 21 abstützt.

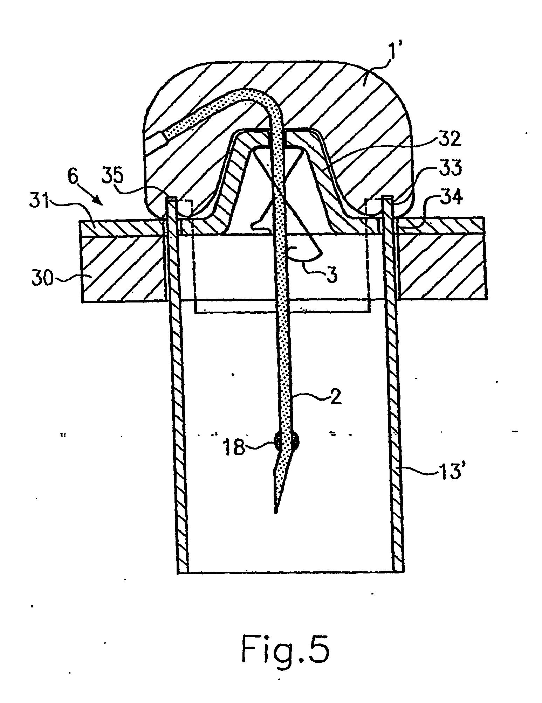

Fig. 5 zeigt eine Ausgestaltung in Verbindung mit einer Huber-Nadel 2, die mittels eines abgebogenen Abschnitts in einem Nadelhalter 1' gehalten ist und zum senkrechten Einfuhren bei einer Injektion vorgesehen ist Mit 30 ist ein vorzugsweise aus Schaumstoffmaterial bestehendes Auflageteil bezeichnet, das mit einer Klebefläche zur besseren Fixierung auf der Haut des Patienten versehen ist. Zwischen dem Auflageteil 30 und dem Nadelhalter 1' ist ein schildförmiges Griffteil 6 angeordnet, das über einen flanschartigen Bereich 31 auf dem Auflageteil 30 aufliegt und sich über einen topfförmigen Mittelabschnitt 32 in eine entsprechende Vertiefung des Nadelhalters 1' erstreckt. In diesem topfförmigen Mittelteil 32 ist das Schutzelement 3 angeordnet.

Beim Herausziehen der Nadel wird das Griffteil 6 auf dem Auflageteil 30 gehalten, während der Nadelhalter 1' abgezogen wird. Hierbei wird das Schutzelement 3 zur Nadelspitze verschoben, bis es an der Nadelkröpfung 18 zum Anliegen kommt, während gleichzeitig die beiden sich kreuzenden Arme des Schutzelementes 3 die Nadelspitze übergreifen und abdecken. Hierauf kann das Griffteil 6 vom Auflageteil 30 oder zusammen mit diesem abgenommen werden. Griffteil 6 und Auflageteil 30 können über eine Klebeschicht auch miteinander verbunden sein.

Die Seitenwände des topfförmigen Mittelteils 32 sind vorzugsweise kegelförmig geneigt, so dass das Griffteil 6 selbst nicht abgezogen, sondern nur gedrückt werden kann.

Fig. 5 zeigt eine Naclelkappe, 13' mit einem rohrförmigen Abschnitt, an dessen proximalem Ende diametral gegenüberliegende Wandabschnitte 33 vorstehen, die über teilkreisförmige Schlitze 34 in dem Flansch 31 des Griffteils 6 in entsprechend teilkreisförmige Nuten 35 im Nadelhalter 1' eingesteckt sind. Die gebogenen Wandabschnitte 33 sind lose durch die gebogenen Schlitze 34 im Flansch 31 des Griffteils 6 geführt und mit Preßsitz in die Nuten 35 des Nadelhalters 1' eingesteckt.

Die Nadelkappe 13' in Fig. 5 kann an der distalen Seite geschlossen ausgebildet sein.

Bei allen Ausführungsformen wird als Schutzelement 3 bevorzugt ein Nadelclip aus Metall verwendet, dessen sich kreuzende Arme von gegenüberliegenden Seiten eines proximalen Wandabschnitts ausgehen, der ein Loch für den Durchtritt der Nadel aufweist, wobei der Lochdurchmesser kleiner ist als die maximale Querabmessung der Nadel an der Quetschung 18, so dass der Nadelclip durch den im Durchmesser vergrößerten Abschnitt 18 in der Schutzstellung an der Nadelspitze gehalten wird. Die sich kreuzenden Arme, die beiderseits der Nadel 2 verlaufen, weisen am distalen Ende einen etwa auf die Breite der Rückwand verbreiterten Endabschnitt auf, der in der Ausgangsstellung auf dem Außenumfang der Nadel unter elastischer Vorspannung aufliegt und bei Erreichen der Nadelspitze durch Federwirkung in die Schutzstellung bewegt wird, in der die beiden verbreiterten Endabschnitte die Nadelspitze übergreifen. Hierzu sind die distalen Enden der Arme, wie die Seitenansichten zeigen, in Längsrichtung etwas versetzt zueinander bzw. die Arme unterschiedlich lang, so dass sichergestellt ist, dass die beiden abgewinkelten Endabschnitte der Arme die Nadelspitze übergreifen. Zumindest am längeren Arm ist der Endabschnitt am freien Rand nach innen gebogen, um die Abdeckung der Nadelspitze auch dann zu gewährleisten, wenn versucht werden sollte, den Nadelclip aus der Schutzstellung auf der Nadel zurückzuschieben, wobei sich der nach innen abgebogene Endabschnitt an der Nadelspitze verhakt. Der Nadelclip kann insgesamt sehr kompakt ausgebildet werden und nur etwa 7 mm lang sein.

## Patentansprüche

1. Schutzvorrichtung für eine Injektions- bzw. Infusionsnadel, umfassend
- einen Nadelhalter (1) am proximalen Ende der Nadel,
- eine vorspringende Eingriffseinrichtung (18) am Nadelumfang nahe der Nadelspitze,
- ein Schutzelement (3) für die Nadelspitze, das auf dem Schaft der Nadel verschiebbar ist und federnde Arme aufweist,
- wobei das Schutzelement durch die vorspringende Eingriffseinrichtung (18) der Nadel daran gehindert ist, über die Nadelspitze hinaus verschoben zu werden, und
- wobei ein Griffteil (6) zum Verschieben bzw Halten des Schutzelementes (3) vorgesehen ist, in dessen Hohlraum das Schutzelement (3) aufgehommen ist,
**dadurch gekennzeichnet,**
**dass** das Griffteil (6) das Schutzelement am proximalen Ende derart übergreift, daß es daran gehindert ist, über das Schutzelement (3) und über die Nadelspitze einaus verschoben zu werden,
wobei eine auf dem Nadelschaft verschiebbare Hülse (4) vorgesehen ist, die eine Verschiebung des Schutzelementes (3) über die Nadelspitze hinaus verhindert.

2. Vorrichtung nach Anspruch 1, wobei das Griffteil (6) einen hohlzylindrischen Abschnitt (7) aufweist, der auf dem Nadelhalter (1) geführt ist und einen Kontakt der Finger mit dem Nadelschaft beim Zurückziehen der Nadel verhindert.

3. Vorrichtung nach Anspruch 2, wobei am distalen Ende des hohlzylindrischen Abschnitts ein Schild (8) ausgebildet ist.

4. Vorrichtung nach den vorhergehenden Ansprüchen, wobei am distalen Ende des Nadelhalters (1) radial abstehende Rippen (11) ausgebildet sind, wobei dieser mit Rippen (11) versehene Abschnitt des Nadelhalters (1) einen größeren Durchmesser hat als das Schutzelement (3) und zum Aufstecken einer Nadelkappe (13) dient.

5. Vorrichtung nach Anspruch 4, wobei das Griffteil (6) auf einem zylindrischen Abschnitt (9) axial verlaufende Schlitze (12) aufweist, durch die die Rippen (11) des Nadelhalters (1) radial vorstehen.

6. Vorrichtung nach den vorhergehenden Ansprüchen, wobei das zylindrische Griffteil (6) einen Hohlraum (10) mit offenem Ende zur Aufnahme des Schutzelementes (3) aufweist, das mit zwei gegenüberliegenden fedemden Armen mit abgedunkelten Enden versehen ist.

7. Vorrichtung nach den vorhergehenden Ansprüchen, wobei das Griffteil (6) am proximalen Ende einen Ringkörper (21) aufweist, der radial nach innen abstehende elastische Finger (22) aufweist und über Bügel (20) mit einem zylindrischen Abschnitt (19) verbunden ist, an dem das Schutzelement (3) anliegt.

## Claims

1. Protective device for a hypodermic needle, comprising
- a needle hub (1) at the proximal end of the needle,
- a projecting engaging means (18) at the needle circumference near the needle tip,
- a protective element (3) for the needle tip which is displaceable on the shaft of the needle and has resilient arms,
- wherein the protective element is prevented by the projecting engaging means (18) of the needle from being displaced beyond the needle tip, and
- wherein a grip part (6), provided for displacing or holding the protective element (3), has a hollow space in which the protective element (3) is held,
**characterized in that**
the grip part (6) overlaps the protective element at the proximal end in such a manner that the grip part is prevented from being displaced beyond the protective element (3) and the needle tip,
wherein a sleeve (4), provided displaceably on the needle shaft, prevents displacement of the protective element (3) beyond the needle tip.

2. Device according to claim 1, wherein the grip part (6) has a hollow cylindrical portion (7) which is guided on the needle hub (1) and prevents contact of the fingers with the needle shaft on withdrawal of the needle.

3. Device according to claim 2, wherein at the distal end of the hollow cylindrical portion a shield (8) is formed.

4. Device according to one of the preceding claims, wherein at the distal end of the needle hub (1), radially protruding ribs (11) are formed, wherein this portion of the needle hub (1) provided with ribs (11) has a larger diameter than the protective element (3) and serves for attaching a needle cap (13).

5. Device according to claim 4, wherein the grip part (6) on a cylindrical portion (9) has axially extending slits through which the ribs (11) of the needle hub (1) radially protrude.

6. Device according to the preceding claims, wherein the cylindrical grip part (6) has a hollow space (10) with an open end for receiving the protective element (3) which is provided with two opposite resilient arms having bent ends.

7. Device according to the preceding claims, wherein the grip part (6) has at its proximal end an annular body (21) which has elastic fingers protruding radially in an inward direction, and is joined by brackets (20) to a cylindrical portion (19) on which the protective element (3) abuts.

## Revendications

1. Dispositif de protection d'une aiguille pour injection ou perfusion, comprenant :
- un support (1) d'aiguille à l'extrémité proximale de l'aiguille,
- un élément de coopération en saillie (18) à la périphérie de l'aiguille proche de la pointe de l'aiguille,
- un élément de protection (3) pour la pointe de l'aiguille qui est coulissant sur la tige de l'aiguille et présente des bras résilients,
- l'élément de protection étant empêché par l'élément de coopération en saillie (18) de l'aiguille d'être déplacé sur la pointe de l'aiguille et
- une partie formant poignée (6) étant prévue pour faire coulisser et maintenir l'élément de protection (3), dans une cavité de laquelle l'élément de protection (3) est reçue, **caractérisé en ce que**
la partie formant poignée (6) déborde de l'élément de protection à l'extrémité proximale, de telle sorte qu'elle est empêchée d'être déplacée sur l'élément de protection (3) et sur la pointe de l'aiguille,
dans lequel une gaine (4) coulissant sur la tige d'aiguille est prévue, qui empêche un déplacement de l'élément de protection (3) sur la pointe de l'aiguiller.

2. Dispositif selon la revendication 1, dans lequel la partie de poignée (6) présente une section cylindrique creuse (7) qui est guidée sur le support (1) d'aiguille et empêche ainsi un contact du doigt avec la tige de l'aiguille lors du retrait de l'aiguille.

3. Dispositif selon la revendication 2, dans lequel, à l'extrémité distale de la section cylindrique creuse, est formée une protection (8).

4. Dispositif selon les revendications précédentes, dans lequel, à l'extrémité distale du support (1) d'aiguille, sont formées des nervures (11) distantes radialement, cette section du support (1) d'aiguille qui est dotée de nervures (11) présentant un diamètre plus grand que celui de l'élément de protection (3) et servant à mettre un capuchon (13) d'aiguille.

5. Dispositif selon la revendication 4, dans lequel la partie formant poignée (6) présente, sur une section cylindrique (9), dcs encoches (12) s'étendant axialement, à travers lesquelles les nervures (11) du support (1) d'aiguille font saillie radialement.

6. Dispositif scion l'une des revendications précédentes, dans lequel la partie cylindrique formant poignée (6) présente une cavité (10) avec une extrémité ouverte pour recevoir l'élément de protection (3), qui est doté de deux bras résilients opposés ayant des extrémités pliées.

7. Dispositif selon l'une des revendications précédentes, dans lequel la partie formant poignée (6) présente à l'extrémité proximale un corps annulaire (21) qui présente des doigts (22) élastiques distants radialement vers l'intérieur et qui est relié par une bride (20) avec une section cylindrique (19) sur laquelle repose l'élément de protection (3).
